# EUROPEAN PATENT APPLICATION

(11) **EP 3 919 040 A1**
(43) Date of publication of application: **08.12.2021**
(21) Application number: 20177761.2
(22) Date of filing: 02.06.2020
(51) Int. Cl.: A61J 1/06, A61J 1/14, A61J 1/20, A61M 5/24, A61M 5/31

(54) **A CARTRIDGE COMPRISING A FILL PORT, A METHOD FOR FILLING THE CARTRIDGE AND AN INJECTION OR INFUSION DEVICE COMPRISING THE CARTRIDGE**

(71) Applicant: Ypsomed AG, 3401 Burgdorf (CH)
(72) Inventor: Burren, Stefan, 3150 Schwarzenburg (CH); Mellenberger, Andres, 3425 Koppigen (CH); Bernhard, Mario, 3400 Burgdorf (CH); Schenker, Susanne, 4912 Aarwangen (CH); Tschirren, Markus, 3400 Burgdorf (CH)
(74) Representative: Dirix, Yvo

(57) **Abstract**

A cartridge for a medicament comprising a barrel defining a longitudinal axis and having an inner volume for receiving the medicament. The barrel having an open end adapted to receive a moveable piston for closing the cartridge, and having an end opposite to the open end forming a fluid outlet for the cartridge for discharging the medicament out of the barrel whereby the fluid outlet is closed by a first pierceable septum. Furthermore, the cartridge comprises a passage forming a fluid inlet port for filling the medicament into the barrel whereby the passage is closed by a second pierceable septum.

## Description

### FIELD OF THE INVENTION

The current invention relates to a cartridge comprising a fill port, a method for filling the cartridge and an injection or infusion device comprising the cartridge.

### BACKGROUND OF THE INVENTION

Fluid medicaments are contained in a primary packaging and often delivered using a delivery device comprising a delivery mechanism to control the delivery rate of the medicament from the primary packaging. The delivery device may be used by a health care professional (HCP) for patient treatment or the patient may use the delivery device for self-medication. Such a delivery device may be an infusion device such as an insulin pump or a patch pump or an injection device such as an injection pen or a bolus injector. Accurate dose setting and dose delivery in terms of delivery volume and delivery rate may be crucial factors for a successful therapy.

The primary packaging may be a medicament container and such containers may be filled with a fluid medicament in a fill finish line under aseptic conditions to preserve sterility of the medicament and prevent contamination. Such containers may be included in the delivery device to form a "ready-to-use" device for the HCP or the patient.

For medicaments that are not long-term stable as a fluid formulation, the solid medicament must be dissolved or reconstituted just before use. Additionally, medicaments may have to be delivered as a fresh emulsion or freshly prepared microemulsion comprising microspheres or microparticles. In this case an empty medicament container, or a medicament container containing a solid medicament is provided together with a liquid medicament or a solvent to prepare the fluid medicament in-situ just before use.

The medicament container may be a rigid container such as a glass cartridge or a cartridge made from a rigid polymer which is closed by a moveable piston or plunger for delivery of the fluid medicament towards a fluid outlet. Alternatively, the medicament container is made from a flexible material which may be compressed or emptied using a suction pump for delivery towards the outlet.

A rigid container such as a glass cartridge has the advantage of accurate dose delivery, a reduced dead volume and enhanced stability of the fluid medicament due to the inertness of the glass. Additionally, rigid containers may have standard dimensions such that they can easily be filled in existing fill finish lines without further modifications. A container made from a flexible material may have a compact design or smaller volume due to the thin flexible walls. However such flexible containers are often non-standardized and require dedicated filling equipment and require more complicated delivery mechanisms.

The medicament container that is filled just prior to use requires at least one fluid inlet port allowing entrance of a fluid medicament or a solvent and requires at least one fluid outlet port for delivery of the medicament into the patient using the delivery mechanism. The inlet port and outlet port may be combined in a single unit or there may be a separate inlet and outlet.

In EP1017317B1 a blood sampling system is presented comprising a syringe as a container made from a rigid material having a separate inlet port and outlet port both located at the distal end wall of the syringe. A fluid (blood) flows into the syringe via the inlet port using an infusion line and the blood sample is expelled via the outlet port. Both ports are connectable to connectors and tubing and are not covered by a septum to avoid the risk of coring.

In WO13184763 an infusion device is presented for fluid delivery comprising a cartridge comprising a collapsible reservoir made from a flexible material. A fluid (insulin) is filled into the collapsible reservoir via a fill port that is closed by a septum. The fluid is delivered via a fluid dispense port (not closed by a septum) using a suction pump system.

In EP2455126A1 a medicament container is presented with flexible walls and having a connection port for fluid delivery and an inlet port for filling the medicament container. The connection port is sealed by a septum. The collapsible container is emptied using a suction pump system.

It is an objective of the present invention to overcome the drawbacks in the prior art and provide a versatile and easy to use cartridge for a fluid comprising a rigid barrel having fluid inlet and outlet port closed by a pierceable septum. Furthermore, it is an objective to provide a method for filling the cartridge and an injection or infusion device adapted to receive the cartridge with the fluid inlet port. Finally a kit of parts is presented comprising the cartridge.

Those objectives are solved by the independent claims.

### DEFINITIONS

The term "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The distal end or distal direction is defined by the direction of the needle configured to penetrate the skin of the patient. For an injection pen this may be the injection needle and the end of the pen holding the needle or being configured to hold the needle is the distal end. For an infusion device the distal end and the distal direction is towards the needle configured to penetrate the skin of the patient, which may be along the axis of the device or tilted or perpendicular to the axis of the device. The distal direction in an infusion device represents the direction in which the medicament flows towards the skin insertion needle. The proximal direction or end is opposite to the distal direction or end.

The word "piston" or "plunger" may be used both as a means for closing the cartridge in a fluid tight manner. For example, the piston may be a cylindrical shaped plug made of an elastomeric material closing a cylindrical shaped cartridge on one end.

In the claims, the word "comprising" does not exclude other elements or steps, and the indefinite article "a" or "an" does not exclude a plurality. For example "passage" or "septum" does not exclude the fact that there may be two "passages" or "septums" that functionally or structurally fulfill the purpose of "a passage" or "a septum".

The wording "barrel" within the context of this application is understood to be a rigid barrel, or a barrel made from a rigid material.

### GENERAL DESCRIPTION OF THE INVENTION

The current invention relates to a cartridge for a medicament comprising a barrel defining a longitudinal axis and having an inner volume for receiving the medicament. The barrel has an open end adapted to receive a moveable piston for closing the cartridge, and an end opposite to the open end forming a fluid outlet for the cartridge for discharging the medicament out of the barrel. The fluid outlet is closed by a first pierceable septum and the cartridge further comprises a passage forming a fluid inlet port for filling the medicament into the barrel whereby the passage is closed by a second pierceable septum. The barrel of the cartridge provides for mechanical strength to the cartridge and is preferably cylindrically shaped and the axis of the cylinder defines the longitudinal axis. Optionally, the barrel has an elliptical cross section. The mechanical strength supports the fill port when the fill port is used for filling the cartridge and supports the first pierceable septum when the connection is established for fluid delivery via the first pierceable septum. Additionally a rigid barrel ensures an accurate control of the medicament delivery rate as the hydraulic pressure is increased once the moveable piston is moved towards the outlet.

The passage in the barrel is configured to establish a connection between the inner volume and the exterior. A fluid, a solvent (aqueous or organic) or a fluid pharmaceutical composition may be filled into the cartridge via the passage. The passage is closed by the second pierceable septum sealing the cartridge and the second septum may be pierced by a needle of a filling device. The first and second pierceable septums seal the cartridge to form an air/liquid tight barrier preventing contamination of the medicament present in the cartridge during shelflife, or to maintain sterility inside of an empty cartridge. The fluid inlet port may be beneficial for medicaments that need to be mixed just prior to use and where liquid formulations are not sufficiently stable over time (e.g. due to particulate formation or denaturation of large molecules). A solvent may be added to dissolve or activate the medicament prior to use, or a fresh emulsion may be formed that would not be stable over time. The fluid inlet port thus increases the versatility of the cartridge as it enables prefilled cartridge applications as well as applications where the medicament is made in-situ or reconstituted just before application of a device comprising the cartridge.

The surface of the first pierceable septum and/or the surface of the second pierceable septum facing the exterior may be sterile and covered by a protective membrane, for example a porous Tyvek membrane. The protective membrane ensures that the surface of the septums remains sterile and may be removed from the surface prior to piercing the first septum or the second septum. The membrane may be removed simultaneously with another part such as a release liber or a cover for a device.The passage of the fluid inlet port for the cartridge defines a second axis that is oriented essentially perpendicular to the longitudinal axis of the barrel. The passage is oriented perpendicular to the longitudinal axis to create space for the fill port and to ensure that the cartridge can be filled sideways.

The barrel of the cartridge preferably comprises a shoulder section and the fluid inlet port is preferably part of the shoulder section. The shoulder section is positioned along the longitudinal axis between the first pierceable septum and the open end of the cartridge. The shoulder section preferably has a reduced dimension to accommodate the outlet for delivery of the medicament. For example a reduced diameter in the shoulder section provides space for the fill port such that the outer dimensions of the cartridge with the fill port do not go beyond the outer dimensions, preferably the diameter, of the barrel. The shoulder section may be strengthened for example by having a higher wall thickness or by ribs, fins or wings radially extending from the shoulder section and those extensions go not beyond the outer dimensions of the barrel. The wings or ribs may additionally be used for guidance and/or rotationally and/or axially fixation of the cartridge in a cartridge holder.

The shoulder section is shaped as a rejuvenation of the barrel to create space for the fill port such that the dimensions of the fill port do not go beyond the dimensions of the barrel. This will keep the design of the cartridge with the fill port compact and ensures that standard cartridge holders may be used for a device comprising the cartridge. Additionally, the cartridge dimensions allow for standard tubs and facilitate tub nesting for fill finish lines accordingly.

The shoulder section of the cartridge comprises an outlet that is closed by the first pierceable septum such that a fluid can be dispensed through the outlet once the first septum has been pierced. The cartridge comprises thus a fill port forming a fluid inlet separate from a fluid outlet for fluid delivery.

The first pierceable septum is attached or connected to the shoulder section of the cartridge closing the outlet of the cartridge, preferably using a crimp and the surface of the first septum facing to the exterior is oriented perpendicular to the longitudinal axis of the barrel. The surface of the second septum is preferably oriented parallel to the axis of the barrel. Fluid is delivered through the outlet of the reservoir and the first septum may be pierced to establish a fluid connection between a medicament present in the inner volume and the exterior. The fluid path may have one needle or cannula for piercing the first septum of the cartridge and one needle or soft cannula for delivery to the patient. The two needles may be connected by a flexible tubing, alternatively, a rigid tubing is used comprising two sharpened ends. A hollow steel tube may be used with two sharpened endings and the steel tube may be straight or bent, for example forming a U-shaped needle.

The cartridge may furthermore comprise the moveable piston for moving along the longitudinal axis and for closing the open end of the cartridge thereby enclosing a first inner volume between the piston and the first pierceable septum that is below the inner volume of the barrel. The piston is moveable within the barrel between a position where there is no liquid enclosed in the volume defined by the barrel, the piston, the first pierceable septum and the fill port, and a position where there is liquid present in the barrel. The piston is reversibly moveable along the axis of the barrel between the two positions and moved in one direction when liquid is filled through the fill port into the cartridge and in the opposite direction when liquid is expelled via the outlet through the first pierceable septum (when this septum has been pierced). The cartridge with the piston, the barrel, the fill port and the first and second pierceable septum form a sterile enclosure before fluid has been filled through the fill port.

Preferably, the fluid inlet port is located between the piston and the first pierceable septum. And thus, the shoulder section comprising the fluid inlet port is located between the piston and the first pierceable septum.

The cartridge for the medicament wherein the piston is positioned in a first position within the barrel which is adjacent or close to the first and/or second pierceable septums when there is no fluid in the cartridge thereby forming the first inner volume within the cartridge.The first inner volume preferably represents a cartridge without a fluid present in the first inner volume. Optionally, only a solid medicament is present within the first inner volume and such solid may be dissolved by the fluid entering through the fill port. The first inner volume is a small volume due to the adjacency of the piston to the first pierceable septum, and may be formed by the outlet or an outlet section of the cartridge comprising the first pierceable septum. Additionally, the volume of the passage oriented perpendicular to the longitudinal axis and closed by the second pierceable septum may contribute to the first inner volume. A low inner first volume has the advantage that a low amount of air or gas is trapped in the cartridge when fluid has been filled through the fill port.

Optionally, the cartridge comprises an air bubble trap.

The cartridge for a medicament wherein the piston is positioned in a second position within the barrel which is adjacent or close to the open end (configured to receive the piston) thereby enclosing a second inner volume that is above the first inner volume when a fluid has been received in the cartridge.

The first and second inner volumes are below the inner volume of barrel. When the piston is positioned in the second position, fluid is present in the cartridge. During filling of the cartridge, the piston is moved along the axis of the barrel from the first position to the second position preferably due to the hydraulic fluid pressure. When fluid is delivered, the piston is moved from the second position towards the first position and can be stopped at intermediate positions if only a part of the fluid needs to be delivered. The piston is moveable from the first position towards the second position during filling and can also be stopped at intermediate positions, for example if a cartridge is only half filled or if two different fluids are subsequently filled through the fluid fill port.

The second pierceable septum of the fluid inlet port of the cartridge is pierceable by a needle of a filling device and the fluid is transferable via the needle of the filling device into the inner volume, preferably initially the first inner volume of the cartridge via the fluid inlet port such that the piston is moved from the first position to the second position as fluid is received (finally forming the second inner volume).The fluid may be a solvent for a solid medicament, for example water to create an aqueous solution. Alternatively, organic solvents may be used or water/oil mixtures to create dispersions or emulsions.

The barrel of the cartridge is preferably made from a rigid polymer, preferably polypropylene or a cycloolefinic copolymer and the wall thickness is adapted to form a stable, non-elastically deformable barrel. Alternatively, the cartridge is at least partially made from glass. Using a rigid polymer has the advantage that the cartridge can be made with a high degree of dimensional accuracy (for example using injection molding). The polymer is preferably a transparent polymer such that the user can see the content within the cartridge. Using polypropylene or a cycloolefinic copolymer has the advantage that biocompatible grades are available certified according to ISO 10993. The surface of the barrel of the cartridge may be coated with a lubrication agent such as silicone oil or, alternatively a fluoro based coating may be chemically adhered or connected to the surface. Using injection molding of a polymer has the advantage that 2-component injection molding techniques may be used to produce the barrel of the cartridge with at least one of the two pierceable septums.

Preferably, the cartridge is not made from a flexible material to create a collapsible reservoir.

The barrel of the cartridge is preferably cylindrically shaped and made from a rigid material having one open end for receiving the piston and an opposite open end comprising a shoulder section for holding the first and/or second pierceable septums. Optionally, the medicament in the cartridge is present as a solid medicament in the first inner volume. The solid medicament may be a lyophilisate. A bulk solution comprising the active ingredient may be filled into the open end of the barrel (the opposite end being closed by the first and second pierceable septums) and a porous cake is formed in the barrel during lyophilisation. In a second step, the piston is positioned in the first position via the opening of the barrel, preferably using a piston positioning tube. The solid medicament may be dissolved in the fluid when fluid is filled into the cartridge and when the piston is moved towards the second position. An inert gas may be filled into the cartridge as well. Using a solid medicament may be advantageous for those medicaments where the long-term stability of the solution is not given. A fresh solution (reconstituted) of the medicament needs to be made each time just prior to delivery.

Optionally, the fluid filled though the fluid fill port comprises the medicament. In this case no medicament is present in the empty cartridge and the fluid medicament in the form of a liquid formulation is filled through the fill port. Alternatively, the final composition of the medicament is formed by combining a component (either a solid or liquid medicament already present in the cartridge) with a liquid (either a solvent or formulation containing (part of) the medicament). The medicament may thus be formed by dissolution and/or chemical reaction and/or by forming a dispersion within the cartridge.

Furthermore, a method for filling the cartridge is presented comprising the steps of:
- providing a cartridge described above,
- providing a filling device comprising the fluid and the fluid is fluidly connected or connectable to a needle of the filling device,
- piercing the second pierceable septum of the fluid inlet port with the needle of the filling device,
- transferring the fluid from the filling device into the cartridge thereby moving the piston from the first position to the second position, followed by
removing the needle of the filling device from the second pierceable septum.

Another aspect of the invention relates to an injection or infusion device comprising the cartridge with the fluid fill port, a housing, a cartridge holder for holding the cartridge, a fluid path comprising a fluid path needle or cannula for piercing the first pierceable septum of the cartridge and a fluid delivery mechanism for advancing the piston in the cartridge to expel fluid through the outlet. The housing may be attachable to the skin of the patient for a patch injection or infusion device and the housing may comprise a flat bottom surface, optionally anatomically shaped. The cartridge is preferably oriented with its longitudinal axis oriented parallel to the flattened bottom surface such that the axis of the passage of the fluid fill port is oriented perpendicular to the bottom surface for sideways filling.

The injection or infusion device may comprise a cartridge holder for holding the cartridge, a fluid path comprising a needle or cannula for piercing the first septum and a delivery mechanism for moving the piston from the second to the first position. Alternatively, the delivery mechanism may be operated in a reversed mode whereby the delivery mechanism moves the piston from the first to the second position to facilitate filling of the liquid through the fill port. In the latter case, the piston in the cartridge may be coupleable to the delivery mechanism that is operated in reverse mode.

The injection or infusion device wherein the housing surrounds at least the inlet port of the cartridge and wherein the housing comprises a housing inlet port that is aligned with the fluid inlet port, preferably with the passage of the fluid inlet port of the cartridge. The housing inlet port is designed as a passage in the housing leaving access to the fluid inlet port of the cartridge. The passage in the housing may be part of a main housing or be part of a cover that is adapted to close the housing once the cartridge has been inserted into the injection or infusion device. The passage in the housing may be closed by a film forming a barrier, for example a Tyvek film, and the film may be removed for accessing the housing inlet port or the needle of the filling device pierces through the film prior to entering the fluid inlet port and piercing the second septum. The passage in the housing thus facilitates the filling of the cartridge when the cartridge is already present in the housing.

The invention further involves a kit of parts comprising at least the injection or infusion device and a filling device comprising a needle for transferring the fluid to the cartridge via the inlet port. Optionally, the kit comprises a container with a solvent or a fluid or solid composition comprising the medicament.

### BRIEF DESCRIPTION OF THE DRAWINGS

The subject matter of the invention will be explained in more detail in the following text with reference to preferred exemplary embodiments which are illustrated in the attached drawings, showing:
- Figure 1:: Cartridge with a fluid inlet port according to the present invention: 3-Dimensional view,
- Figure 2:: Barrel for cartridge with a fluid inlet port: Cross section without a piston,
- Figure 3:: Cartridge with a fluid inlet port according to the present invention with the first and second pierceable septums: Cross section without a piston,
- Figure 4:: Cartridge with a fluid inlet port according to the present invention: Cross section with a piston in first position,
- Figure 5:: Cartridge with a fluid inlet port according to the present invention: Cross section with a piston in second position,
- Figure 6:: Top view of cartridge with fluid inlet port and fluid exit port,
- Figure 7:: Detail fluid inlet port,
- Figure 8:: Injection device comprising the cartridge,
- Figure 9:: Cross section injection device,
- Figure 10:: Detail of the device presented in Figure showing the fluid inlet port and the housing inlet port.

### DETAILED DESCRIPTION OF PREFERRED EMBODIMENTS

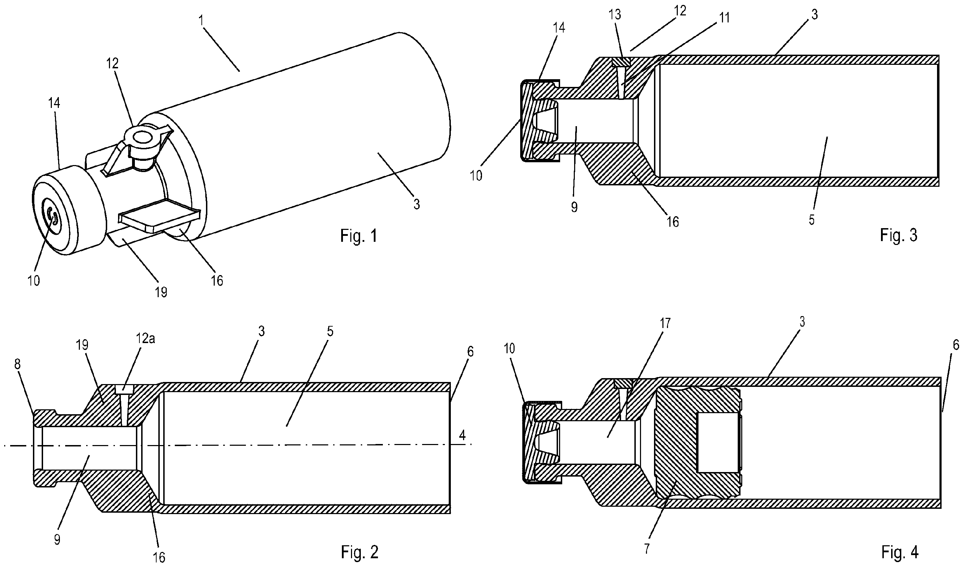

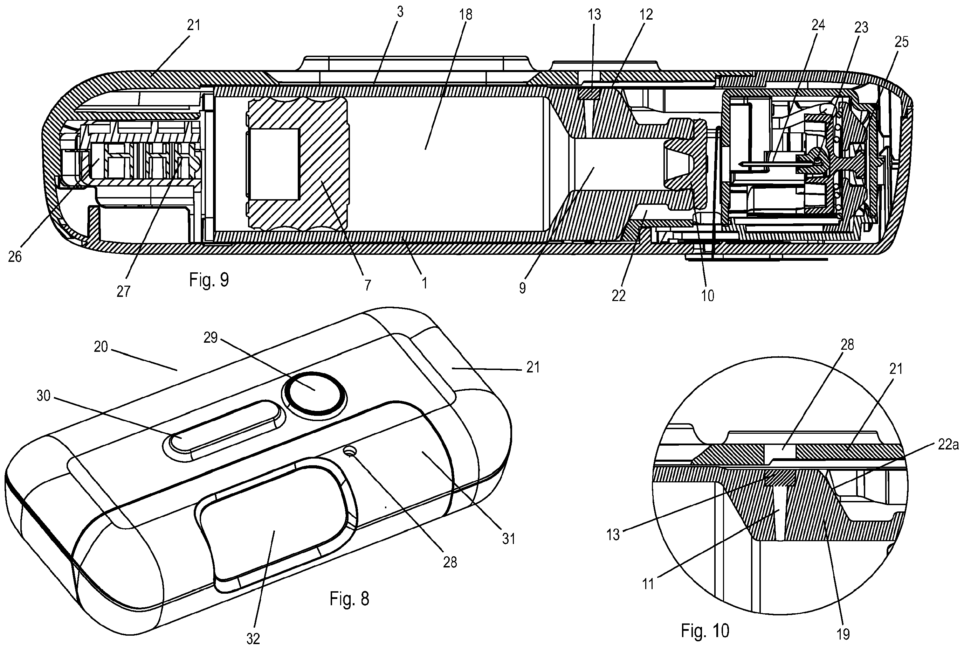

A cartridge according to the present invention is shown in Figures 1 to 7 and an injection device comprising the cartridge in Figures 8 to 10.

Empty cartridge: The cartridge 1 comprises a barrel 3 which in this example is a cylinder with a circular cross section. Alternatively, the cross section is elliptical to save space in one dimension for a more flat design of an injection device comprising the cartridge. The barrel 3 has a longitudinal axis 4 and defines an inner volume 5 extending from an open end 6 to an end opposite to the open end (Figure 2). The opposite end 8 comprises a fluid outlet 9 formed as a rejuvenation of the barrel 3 and being part of a shoulder section 16 of the cartridge 1. The opposite end 8 is closed by a first pierceable septum 10 which may be attached to the cartridge 1 using a crimp 14 (Figure 3). The first pierceable septum 10 may be pierced by a fluid path needle to establish a fluid connection between the cartridge and a fluid path. Optionally, the shoulder section 16 is strengthened, for example with a plurality of fins or radial extensions 19. The fins or extension may also be used the position or fixate the cartridge 1 within a cartridge holder.

The barrel 3 of the cartridge as displayed in Figure 2, optionally including the fins 19, the outlet 9 and the shoulder section 16, may be made from a rigid polymer as a single unit, for example using injection molding. The polymer may be selected from a cycloolefinic (co-)polymer (COC or COP), a polycarbonate, polystyrene or polypropylene. Preferably, the barrel is made from a transparent poymer such that the user can see the contents or degree of fluid filling in the cartridge. Alternatively, the barrel of the cartridge is made from glass. The inside surface of the barrel may be coated with a lubricant to facilitate movement of a piston. For example a silicone oil may be used as the lubricant.

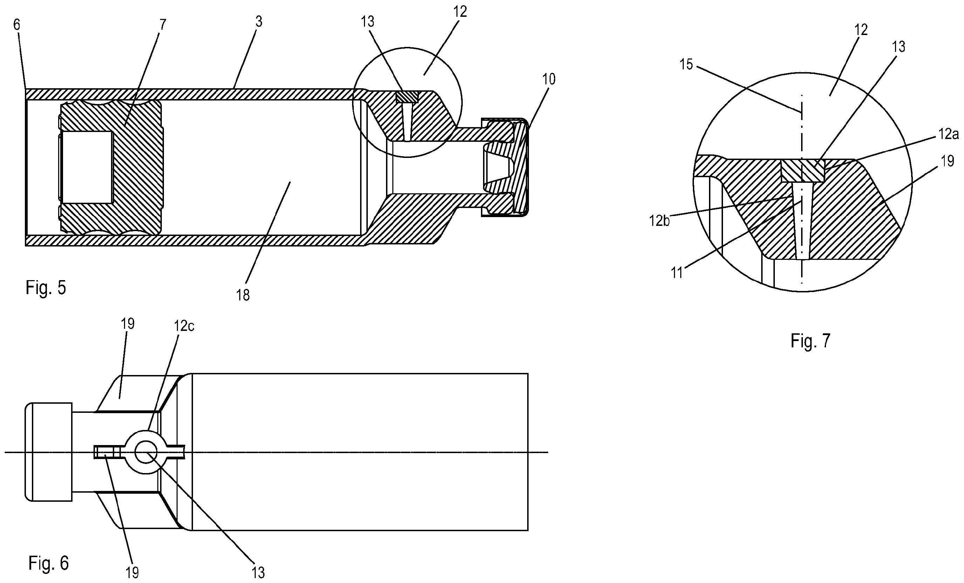

The cartridge 1 comprises a fluid inlet port 12 located adjacent to the fluid outlet 9 of the cartridge 1 and the fluid inlet port 12 is preferably part of the shoulder section 16 (Figure 3). The fluid inlet port 12 comprises a passage 11 connecting the inner volume 5 of the barrel with the exterior. Preferably, the passage 11 connects the exterior with the fluid outlet 9. The passage 11 has a longitudinal axis that is preferably orthogonal or optionally inclined with the longitudinal axis 4 of the barrel. The passage 11 may have a cone shaped section 12b to facilitate guidance of a needle of a filling device (Figure 7). The fluid inlet port 12 is closed by a second pierceable septum 13 which may be pierced by a needle of a filling device for filling the cartridge 1. The first and second pierceable septums 10,13 may reseal after a needle has been removed and the first and second pierceable septums provide a barrier between the exterior and the interior of the cartridge and protect an inner volume or a medicament present in the cartridge from contamination. The first and second pierceable septums 10,13 are preferably made from an elastomeric material such as a rubber. The rubber may be based on a natural rubber or a synthetic rubber such as butyl rubber or silicone rubber or a thermoplastic elastomer (TPE). Optionally, the fluid inlet port 12 is part of the fin or radial extension 19 to provide mechanical support to the fluid inlet port. Preferably, the fluid inlet port 12 is located in the shoulder section 16 or a rejuvenated region of the barrel such that the fluid inlet port radially extends from the longitudinal axis 4 of the barrel without going beyond the outer dimensions of the barrel for obtaining a compact cartridge design. Optionally, the fluid inlet port radially extends beyond the outer dimensions of the barrel to facilitate access to the fluid inlet port 12 once the cartridge 1 has been inserted in an injection device.

The second pierceable septum 13 is fixated to the cartridge 1 to provide a fluid tight barrier between the barrel 3, or the shoulder section 16 of the barrel and the septum 13. The fixation is such that the septum 13 withstands the mechanical forces acting on the septum during penetration or the fluid pressure during filling of the cartridge. The fixation of the second pierceable septum 13 may be by mechanical fixation, for example the pierceable septum 13 is press-fitted into a recess 12a surrounding the passage 11 of the fluid inlet port 12 (Figures 2 and 7). Optionally the septum 13 has a radially and/or axially extending rim engaging a second recess present in the recess 12a. Alternatively, the walls of the recess 12a may have a threading engaging an external threading of the septum 13. Optionally, and additionally, the septum 13 may be adhesively connected to the fluid inlet port 12. The second septum 13 may also be attached to the cartridge using a crimp partially enclosing a cylindrical extension 12c (Figure 6) of the fluid inlet port 12. The crimp may be made from a metal and may have a cone shaped opening for guiding an insertion needle towards the septum. The crimp is preferably attached by deformation of the crimp around the cylindrical extension 12c. Optionally, a cap or disc with an entrance hole covers at least partially the septum and is attached to the fill port 12 using an adhesive or ultrasonic welding or laser welding. In another embodiment, the second septum may have a dogbone shape and the two radial extensions fit within two recesses one facing the outside and one facing inside the barrel. Alternatively, the second septum is located not on the outside surface of the barrel but press fitted into a cavity located halfway the passage.

As yet another alternative, the barrel for the cartridge and the second pierceable septum are produced using 2-component injection molding techniques

Cartridge with piston: A cartridge 1 with a moveable piston 7 is shown in Figure 4. The piston 7 has been inserted from the open end 6 into the barrel 3, for example using a positioning tube. The piston 7 has been positioned in a first position close to the outlet 9 and adjacent to the fluid inlet port 12. The inner surface of the first pierceable septum 10, together with the walls of the outlet 9, the rejuvenated section of the barrel and a distal end surface of the piston 7 form a first inner volume 17.

The first inner volume 17 is below the inner volume of the barrel 5. The fluid inlet port 12 is located between the piston 7 and the first pierceable septum 10.

The first inner volume 17 may be empty, e.g. not filled at all, or the first inner volume may be filled with an inert gas such as nitrogen or argon, or the first inner volume may be filled with a liquid comprising the medicament or a precursor of the medicament, or the first inner volume may be filled with a solid medicament, for example a lyophilisate, a powder or (agglomerated) microspheres. Optionally, the first inner volume is subjected to an under-pressure (vacuum). Combinations of the aforementioned options can easily be envisaged by the skilled person without deviating from the current invention, for a example solid medicament may be combined with an inert gas filling.

A cartridge 1 with the moveable piston 7 located at a second position for a filled cartridge 1 is shown in Figure 5. The piston 7 has been moved along the axis 4 from the first position towards the second position. When the piston 7 is in the second position, a second inner volume 18 is enclosed by the walls of the barrel 3, the outlet 9, the fill port 12, the first pierceable septum 10 and the distal end surface of the piston 7. The second inner volume 18 is above the first inner volume 17 but below the inner volume 5 of the barrel. The piston 7 has been moved from the first position to the second position by the pressure of the fluid that has been filled through the fill port 12. The second pierceable septum 13 of the fill port 12 has been pierced by a needle of a filling device and fluid has been transferred from the filling device into the cartridge and the fluid pressure moves the piston along the axis of the barrel. The movement of the piston 7 due to the hydraulic pressure may be assisted by active movement of the piston 7 towards the open end 6, for example by a delivery mechanism connected to the piston 7 and which is operated in a reverse mode.

The open end of the barrel may have an additional stop or rim preventing detachment of the piston 7 from the cartridge 1 through the open end. Such a rim or protrusion may be located at the open end 6 facing towards the center and have a sloped surface (to facilitate entrance of the piston) and an orthogonal surface (to prevent piston removal).

During movement of the piston 7 towards the second position a fluid medicament is introduced into the cartridge or a solvent dissolves the solid or microspheres present in the cartridge.

An injection device 20 comprising the cartridge is presented in Figure 8 showing a housing 21 closed by a cover 31 and having a push button 29 for starting an injection (or infusion) or for starting a needle insertion mechanism. The injection device furthermore comprises an indicator 30 for example using Led lights. The injection device 20 has a flat bottom surface which may be attached to the body by a not shown adhesive layer present at the bottom surface. The housing 21, or optionally the cover 31 comprises a housing inlet port 28 which is designed as a passage in the housing. The housing inlet port 28 may be covered by a protective layer prior to filling of the device. Optionally, the cover 31 has a viewing window 32 for viewing the presence of a cartridge and/or medicament. A cross section of the injection device is shown in Figure 9. The cartridge 1 is located in a cartridge holder 22 located inside the housing behind the cover 31. The cartridge is axially fixated in the cartridge holder by the fin 19 abutting a protrusion 22a located in the cartridge holder or within the housing (Figure 10). Closure of the housing 21 by the cover 31 may thus simultaneously axially and rotationally fixate the cartridge 1. The accurate positioning of the cartridge is important for alignment of the fluid inlet port 12 of the cartridge 1 with the housing inlet port 28. The passage in the housing may have an elongated shape or have a larger dimensions to correct for play between the cartridge 1 and the cartridge holder. Once aligned (Figure 10), the passage of the housing inlet port 28 and the passage of the fluid inlet port 12 are substantially axially aligned such that a needle from an insertion device can penetrate the second pierceable septum 13. The injection device 20 comprises a fluid path needle 24 that is aligned with the first pierceable septum 10 of the cartridge. The fluid path needle 24 can be moved by a fluid path needle insertion mechanism 25 from a retracted position to an inserted position for penetrating the first pierceable septum 10. The fluid path needle insertion mechanism may be biased by a spring which is released for movement of the needle 24 towards the inserted position. The fluid path needle insertion mechanism 25 may be coupled to a skin needle insertion mechanism which is configured to move a skin needle from a position within the housing to a position outside the housing for skin penetration. Preferably the skin needle is oriented perpendicular to the fluid path needle 24. The skin needle and fluid path needle are fluidly coupled by a fluid path such as a tubing. The injection device furthermore has a fluid delivery mechanism 26 which is configured to advance a piston rod 27 into the cartridge for advancing the piston 7. The fluid delivery mechanism may be spring driven or driven by an electric motor. Preferably, the distal end of the piston rod 27 abuts the proximal end of the piston 7. Optionally, the piston rod 27 may be coupled or connected to the piston 7. The fluid delivery mechanism is activated once the fluid path needle and skin needle have been moved from their retracted into the inserted positions such that advancement of the piston 7 from the second position to the first position expels medicament from the device.

Using a cartridge with a rigid barrel and a piston in combination with the fluid delivery mechanism having a piston rod has the advantage of accurate control of the dose delivery rate compared to devices using a collapsible reservoir. Additionally, the delivery mechanism for the rigid cartridge is not directly pumping the fluid out of the cartridge and is therefore less susceptible to leakage.

**LIST OF REFERENCE SIGNS**

| | |
|---|---|
| 1 Cartridge | 17 First inner volume |
| 2 Medicament | 18 Second inner volume |
| 3 Barrel | 19 Fin / Radial extension |
| 4 Longitudinal axis | 20 Injection device |
| 5 Inner volume barrel | 21 Housing |
| 6 Open end | 22 Cartridge holder |
| 7 Moveable piston | 22a Protrusion |
| 8 Opposite end | 23 Fluid path |
| 9 Fluid outlet | 24 Fluid path needle |
| 10 First pierceable septum | 25 Fluid path needle insertion mechanism |
| 11 Passage | 26 Fluid delivery mechanism |
| 12 Fluid inlet port | 27 Piston rod |
| 12a Recess | 28 Housing inlet port |
| 12b Cone shaped passage | 29 Push button |
| 12c Cylindrical extension | 30 Indicator |
| 13 Second pierceable septum | 31 Cover |
| 14 Crimp | 32 Viewing window |
| 15 Second axis | |
| 16 Shoulder section | |

## Claims

1. A cartridge for a medicament comprising:
a barrel defining a longitudinal axis and having an inner volume for receiving the medicament the barrel having an open end adapted to receive a moveable piston for closing the cartridge,
and having an end opposite to the open end forming a fluid outlet for the cartridge for discharging the medicament out of the barrel whereby the fluid outlet is closed by a first pierceable septum,
**characterized by**
that the cartridge comprises a passage forming a fluid inlet port for filling the medicament into the barrel whereby the passage is closed by a second pierceable septum.

2. The cartridge for a medicament according to claim 1 wherein the passage of the fluid inlet port defines a second axis that is oriented essentially perpendicular to the longitudinal axis.

3. The cartridge for a medicament according to claims 1 or 2 wherein the barrel comprises a shoulder section and the fluid inlet port is part of the shoulder section and positioned along the longitudinal axis between the first pierceable septum and the open end of the cartridge.

4. The cartridge for a medicament according to any of the previous claims furthermore comprising the moveable piston for moving along the longitudinal axis and for closing the open end of the cartridge thereby enclosing a first inner volume between the piston and the first pierceable septum that is below the inner volume of the barrel.

5. The cartridge for a medicament according to claim 4 wherein the fluid inlet port is located between the piston and the first pierceable septum.

6. The cartridge for a medicament according to claims 4 or 5 wherein the piston is positioned in a first position within the barrel which is adjacent or close to the first and/or second pierceable septum when there is no fluid in the cartridge thereby forming the first inner volume.

7. The cartridge for a medicament according to claims 4 or 5 wherein the piston is positioned in a second position within the barrel which is adjacent or close to the open end thereby enclosing a second inner volume that is above the first inner volume when a fluid has been received in the cartridge.

8. The cartridge for a medicament according to claim 7 wherein the second pierceable septum of the fluid inlet port is pierceable by a needle of a filling device and whereby the fluid is transferable via the needle of the filling device to the cartridge via the fluid inlet port such that the piston is moved from the first position to the second position as fluid is received in the cartridge.

9. The cartridge for medicament according to any of the previous claims wherein the barrel of the cartridge is made from a rigid polymer, preferably polypropylene or a cycloolefinic copolymer.

10. The cartridge for a medicament according to any of claims 4 to 9 wherein a solid medicament is present in the first inner volume.

11. The cartridge for a medicament according to any of claims 4 to 9 wherein the fluid comprises the medicament.

12. A method for filling a cartridge for a medicament comprising the steps of:
providing the cartridge according to claims 4 to 11,
providing a filling device comprising the fluid that is connected or connectable to the needle of the filling device,
piercing the second pierceable septum of the fluid inlet port with the needle of the filling device,
transferring the fluid from the filling device into the cartridge thereby moving the piston from the first position to the second position
removing the needle of the filling device from the second pierceable septum.

13. An injection or infusion device comprising the cartridge according to claims 4 to 11, comprising a housing, a cartridge holder for holding the cartridge, a fluid path comprising a fluid path needle or canula for piercing the first pierceable septum of the cartridge and a fluid delivery mechanism for advancing the piston in the cartridge

14. The injection or infusion device according to claim 13 wherein the housing surrounds at least the fluid inlet port of the cartridge and wherein the housing comprises a housing inlet port that is aligned with the fluid inlet port of the cartridge.

15. Kit of parts comprising the injection or infusion device according to claims 13 or 14 and a filling device comprising a needle for transferring the fluid to the cartridge via the fluid inlet port.
